# EUROPEAN PATENT SPECIFICATION

(11) **EP 3 549 585 B1**
(45) Date of publication and mention of the grant of the patent: **12.06.2024**
(21) Application number: 17875830.6
(22) Date of filing: 30.11.2017
(51) Int. Cl.: A61K 31/444, A61P 7/02, A61K 47/38, A61K 47/36, A61K 47/32, A61K 47/26, A61K 47/18, A61K 47/12, A61K 9/20, A61K 9/00

(54) **ORALLY DISINTEGRATING TABLET OF EDOXABAN TOSYLATE HYDRATE**
ORAL ZERFALLENDE EDOXABANTOSYLAT-HYDRAT TABLETTE
COMPRIMÉ ORODISPERSIBLE D'HYDRATE DE TOSYLATE D'EDOXABAN

(30) Priority: 01.12.2016 JP 2016234259
(43) Date of publication of application: 09.10.2019
(73) Proprietor: Daiichi Sankyo Company, Limited, Tokyo 103-8426 (JP)
(72) Inventor: KIKKAWA, Yoshito, Tokyo 103-8426 (JP); KATO, Takafumi, Tokyo 103-8426 (JP)
(74) Representative: Marks & Clerk LLP
(86) International application number: PCT/JP2017/042930
(87) International publication number: WO 2018/101373

(56) References cited:
- EP-A1- 2 540 317
- EP-A1- 2 540 318
- EP-A1- 2 742 941
- EP-A1- 2 842 549
- WO-A1-2013/022059
- WO-A1-2013/022924
- WO-A1-2013/161823
- CN-A- 102 058 889
- DOLLY A. PARASRAMPURIA ET AL: "Evaluation of regional gastrointestinal absorption of edoxaban using the enterion capsule", JOURNAL OF CLINICAL PHARMACOLOGY., vol. 55, no. 11, 26 June 2015 (2015-06-26) , pages 1286-1292, XP055705541, US ISSN: 0091-2700, DOI: 10.1002/jcph.540

## Description

### Technical Field

The present invention relates to an orally disintegrating tablet comprising edoxaban, a pharmacologically acceptable salt thereof, or a solvate thereof, the tablet rapidly disintegrating when put in the mouth or when placed in water, and having sufficient hardness in usual production, transportation, and use; and a production method thereof. Both the tablet and the production method of the present invention are further defined in the claims. Any subject-matter outside the scope of the claims is not part of the present invention and provided for comparison or reference only. T

### Background Art

Known dosage forms of oral solid formulations in the fields of pharmaceuticals and foods include tablets, capsules, granules, and powders, but it is desired to develop an orally disintegrating tablet that rapidly disintegrates when put in the mouth or when placed in water as a dosage form that is easier for elderly people, infants, and patients with dysphagia to take.

In addition to the property of rapidly disintegrating in the oral cavity, the orally disintegrating tablet should also have sufficient hardness to withstand physical shocks in production, transportation, and use, as for conventional tablets. Moreover, the orally disintegrating tablet preferably has good taste, with unpleasant taste and stimulation suppressed when put in the mouth, also from the point of view of medication compliance.

Various reports about orally disintegrating tablets have been made so far. For example, Patent Literature 1 describes an orally disintegrating tablet comprising a drug, microcrystalline cellulose with a bulk density of 0.23 g/cm³ or less, a sugar alcohol, and pregelatinized starch. However, this document does not describe an orally disintegrating tablet comprising edoxaban and an organic acid.

Moreover, Patent Literature 2 describes the invention of a levofloxacin-containing tablet comprising levofloxacin and hydroxypropyl cellulose and being excellent in terms of disintegration and suspension into water, but no orally disintegrating tablet comprising edoxaban, an organic acid, and hydroxypropyl cellulose.

Meanwhile, Patent Literature 3 describes the invention of a pharmaceutical composition having improved dissolution properties, wherein the composition comprises (a) edoxaban, a pharmacologically acceptable salt thereof, or a solvate thereof and (b) one or more selected from sugar alcohols and water-swelling additives; and a coated tablet comprising a tablet comprising the aforementioned (a) and (b), and further comprising (c) a water-swelling additive, the tablet coated with a coating at least comprising one or more selected from hypromellose, methyl cellulose, ethyl cellulose, hydroxypropyl cellulose, and polyvinyl alcohol. However, an object of this document is to obtain a pharmaceutical composition comprising edoxaban and having improved dissolution properties, and the document does not describe an orally disintegrating tablet comprising edoxaban and an organic acid and having both high disintegrability and dissolution properties into water.

Furthermore, Patent Literature 4 describes a formulation having improved dissolution properties, comprising (a) edoxaban, a pharmacologically acceptable salt thereof, or a solvate thereof and (b) an organic acid, but no orally disintegrating tablet having both high disintegrability into water and sufficient hardness to withstand physical shocks in production, transportation, and use.

Further oral dosage forms are described in Patent Literature 5-7. An evaluation of the gastrointestinal absorption of edoxaban is described in Non-Patent Literature 1.

### Citation List

### Patent Literature

Patent Literature 1: US Patent application publication No. 2015/110880 (EP2842549)
Patent Literature 2: Japanese Patent No. 5295506
Patent Literature 3: US Patent No. 9149532
Patent Literature 4: US Patent No. 9402907 (EP2742941)
Patent Literature 5: EP2540318
Patent Literature 6: EP2540317
Patent Literature 7: WO 2013/022924

### Non-Patent Literature

Non-Patent Literature 1: Dolly et al., J. Clin. Pharm., vol. 55, 11, 26 June 2015, pages 1286-1292

### Summary of Invention

### Technical Problem

An object of the present invention is to provide an orally disintegrating tablet comprising edoxaban, a pharmacologically acceptable salt thereof, or a solvate thereof, the tablet rapidly disintegrating when put in the mouth or when placed in water, and having sufficient hardness in usual production, transportation, and use, and excellent storage stability.

### Solution to Problem

The present inventors studied diligently in order to solve the problem and have found as a result that an orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (D) one or more disintegrant selected from the group consisting of carmellose, crospovidone, carmellose calcium, croscarmellose sodium, corn starch, sodium starch glycolate, and pregelatinized starch could solve the problem, thereby completing the present invention.

Accordingly, the present invention provides an orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (D) one or more disintegrant selected from the group consisting of carmellose, crospovidone, carmellose calcium, croscarmellose sodium, corn starch, sodium starch glycolate, and pregelatinized starch; and a production method thereof.

Accordingly, the present invention relates to the following (1) to (14)
(1) An orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (D) one or more disintegrant selected from the group consisting of carmellose, crospovidone, carmellose calcium, croscarmellose sodium, corn starch, sodium starch glycolate, and pregelatinized starch.
(2) The orally disintegrating tablet according to (1), wherein (D) the disintegrant is carmellose, crospovidone, and pregelatinized starch.
(3) The orally disintegrating tablet according to (1) or (2), further comprising a sugar alcohol and microcrystalline cellulose having a bulk density of 0.26 g/cm³ or less.
(4) The orally disintegrating tablet according to (3), wherein the sugar alcohol is D-mannitol.
(5) The orally disintegrating tablet according to any one of (1) to (4), wherein the average degree of gelatinization of the pregelatinized starch is 90% or less.
(6) An orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, (E) carmellose, (F) D-mannitol, (G) microcrystalline cellulose having a bulk density of 0.26 g/cm³ or less, and (H) pregelatinized starch.
(7) The orally disintegrating tablet according to (6), comprising: a drug-containing mixed powder or drug-containing granules comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose; and a drug-free mixed powder comprising (F) D-mannitol, (G) microcrystalline cellulose having a bulk density of 0.26 g/cm³ or less, and (H) pregelatinized starch.
(8) The orally disintegrating tablet according to (6), comprising: a drug-containing mixed powder or drug-containing granules comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose; and drug-free granules comprising (F) D-mannitol, (G) microcrystalline cellulose having a bulk density of 0.26 g/cm³ or less, and (H) pregelatinized starch.
(9) The orally disintegrating tablet according to (6), comprising drug-containing granules comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose; and drug-free granules comprising (F) D-mannitol, (G) microcrystalline cellulose having a bulk density of 0.26 g/cm³ or less, and (H) pregelatinized starch.
(10) The orally disintegrating tablet according to any one of (6) to (9), further comprising one or more ingredients selected from the group consisting of crospovidone, carmellose calcium, croscarmellose sodium, corn starch, sodium starch glycolate, and pregelatinized starch.
(11) The orally disintegrating tablet according to any one of (6) to (10), wherein the microcrystalline cellulose is 1 to 50% by weight relative to the total weight of the tablet.
(12) The orally disintegrating tablet according to any one of (6) to (11), wherein the average degree of gelatinization of the pregelatinized starch is 90% or less.
(13) The orally disintegrating tablet according to any one of (6) to (12), wherein the tablet disintegrates within 60 seconds in the "Disintegration Test" in the Japanese Pharmacopoeia 16th edition, wherein the result is the maximum value of 6 tablets.
(14) A method for producing an orally disintegrating tablet, comprising: a step of mixing (A) edoxaban tosylate hydrate, (B) 0.1 to 15% by weight of fumaric acid relative to the total weight of the tablet, and (E) carmellose, and spraying, onto the mixture, (C) 0.1 to 2.0% by weight of hydroxypropyl cellulose relative to the total weight of the tablet, dissolved in water, to produce drug-containing granules; a step of mixing (F) D-mannitol and (G) microcrystalline cellulose having a bulk density of 0.26 g/cm³ or less, and spraying, onto the mixture, (H) pregelatinized starch dissolved or dispersed in water to produce drug-free granules; and a step of compression molding the obtained two types of granules.
(15) A method for producing an orally disintegrating tablet, comprising: a step of mixing (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose, and spraying water onto the mixture to produce drug-containing granules; a step of mixing (F) D-mannitol and (G) microcrystalline cellulose having a bulk density of 0.26 g/cm³ or less, and spraying, onto the mixture, (H) pregelatinized starch dissolved or dispersed in water to produce drug-free granules; and a step of compression molding the obtained two types of granules.

### Advantageous Effects of Invention

The present invention successfully provided an orally disintegrating tablet comprising edoxaban tosylate hydrate, having both rapid disintegrability and solubility when placed in the oral cavity or water, having good taste, having sufficient hardness in the usual process of production, transportation, and use, and excellent storage stability.

Furthermore, the present invention successfully provided a method for producing an orally disintegrating tablet with the aforementioned excellent characteristics by conventional compression molding without requiring a complicated step or special facilities.

### Description of Embodiments

In the present invention, an orally disintegrating tablet is a compression molded product having both rapid disintegrability and solubility when put in the mouth or placed in water. Specifically, the term means a tablet that disintegrates within usually about 5 to 180 seconds, preferably about 5 to 60 seconds, and further preferably about 5 to 40 seconds in a disintegration test in the oral cavity mainly with the saliva, a disintegration test with an apparatus, or the like.

The orally disintegrating tablet according to the present invention has sufficient hardness in the usual process of production, transportation, and use. For example, the tablet is an orally disintegrating tablet having a hardness of usually 2 kg or more, more preferably 3 kg or more, and further preferably 5 kg or more in a hardness test.

The orally disintegrating tablet according to the present invention has dissolution properties suitable for pharmaceutical preparations. For example, the tablet is an orally disintegrating tablet that usually exhibits an average percentage dissolution of 80% or more and preferably exhibits a percentage dissolution of 85% or more at a time point of 30 minutes in a dissolution test.

Edoxaban is N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide, represented by the following formula (1) (hereinafter, also referred to as Compound 1).

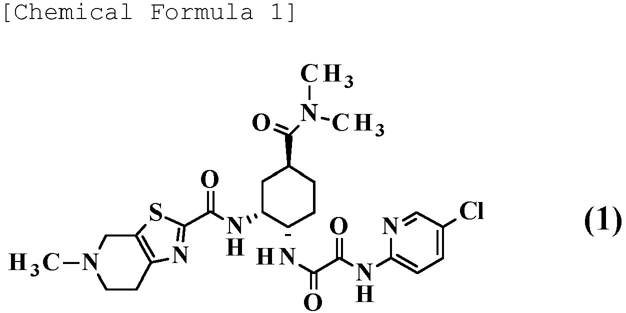

Compound 1 may be a solvate (including hydrate) and may be a pharmacologically acceptable salt or a solvate (including hydrate) thereof. The pharmacologically acceptable salt thereof or solvate used in the present invention is N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (edoxaban tosylate hydrate), represented by the following formula (1a).

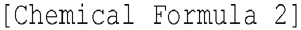

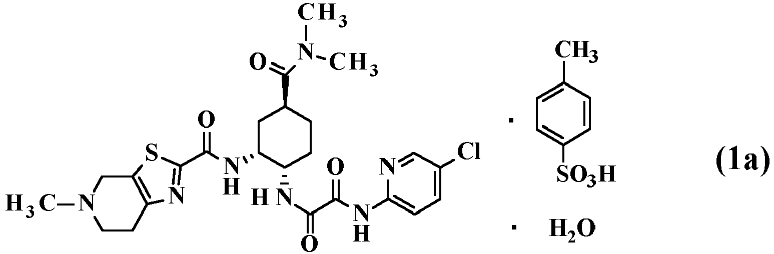

Edoxaban exhibits thrombogenesis-inhibiting activity by selectively, reversibly, and directly inhibiting activated blood coagulation factor X (FXa), which acts to form thrombus by producing thrombin from prothrombin and promoting fibrin formation in the blood coagulation cascade.

Edoxaban has been used in clinical trials conducted in Japan and other countries for inhibition of development of venous thromboembolism in patients who have undergone lower limb orthopedic surgery including total knee replacement, total hip replacement, and hip fracture surgery and for inhibition of development of ischemic stroke and systemic embolism and treatment and suppression of relapse of venous thromboembolism (deep venous thrombosis and pulmonary thromboembolism) in patients with nonvalvular atrial fibrillation.

Edoxaban, in particular edoxaban tosylate hydrate, contained in the orally disintegrating tablet according to the present invention is administered orally once a day for inhibition of development of ischemic stroke and systemic embolism and treatment and suppression of relapse of venous thromboembolism (deep venous thrombosis and pulmonary thromboembolism) in patients with nonvalvular atrial fibrillation usually at a dose of 30 mg (when the body weight is 60 kg or less) or 60 mg (when the body weight is more than 60 kg) in terms of edoxaban for adults. The dose may be reduced to 30 mg once a day depending on renal function and any concomitant drug(s). Moreover, the tablet may be administered orally, usually at a dose of 30 mg in terms of edoxaban once a day for adults, in the inhibition of development of venous thromboembolism in patients who have undergone lower limb orthopedic surgery.

The term "acid" as used herein refers to a compound that is acidic and can be added to pharmaceutical preparations and includes organic acids and inorganic acids.

The term "organic acid" as used herein refers to an organic compound that is acidic and can be used as an excipient for pharmaceutical preparations. Examples of the organic acids include carboxylic acids, sulfonic acids, enols, or salts thereof.

The term "acidic" as used herein refers to the pH (hydrogen-ion exponent) in an aqueous solution being less than 7, and "neutral" refers to the pH being 7.

The term "inorganic acid" used herein is not particularly limited, as long as it is an inorganic acid that can be added to pharmaceutical preparations, but examples thereof include hydrochloric acid or phosphoric acid.

The term "salt of an inorganic acid" used herein is not particularly limited, as long as it is a salt of the inorganic acid that can be added to pharmaceutical preparations, but examples thereof include sodium hydrogen sulfite, potassium dihydrogen phosphate, or sodium dihydrogen phosphate.

The term "carboxylic acid" used herein is not particularly limited, as long as it is a carboxylic acid that can be added to pharmaceutical preparations, but examples thereof include adipic acid, aspartic acid, alginic acid, benzoic acid, carboxyvinyl polymer, carmellose, citric acid, glutamic acid, succinic acid, acetic acid, tartaric acid, sorbic acid, lactic acid, hydroxypropyl methyl cellulose acetate succinate, fumaric acid, maleic acid, malonic acid, anhydrous citric acid, a methacrylic acid copolymer, or malic acid; preferable examples thereof include adipic acid, aspartic acid, alginic acid, carboxyvinyl polymer, carmellose, citric acid, tartaric acid, hydroxypropyl methyl cellulose acetate succinate, fumaric acid, maleic acid, malonic acid, a methacrylic acid copolymer, or malic acid; and more preferable examples thereof include aspartic acid, alginic acid, carmellose, or fumaric acid.

The term "salt of the carboxylic acid" used herein is not particularly limited, as long as it is a carboxylic acid salt that can be added to pharmaceutical preparations, but examples thereof include sodium L-aspartate, sodium benzoate, sodium carboxymethyl starch, carmellose potassium, carmellose calcium, carmellose sodium, sodium dihydrogen citrate, disodium citrate, calcium gluconate, monosodium L-glutamate, croscarmellose sodium, monosodium succinate, calcium acetate, vinyl acetate resin, sodium tartrate, potassium hydrogen tartrate, potassium sorbate, calcium lactate, monosodium fumarate, sodium stearyl fumarate, sodium citrate anhydrous, or sodium malate; preferable examples thereof include sodium carboxymethyl starch, carmellose potassium, carmellose calcium, carmellose sodium, croscarmellose sodium, or sodium stearyl fumarate; and more preferable examples thereof include carmellose potassium, carmellose calcium, croscarmellose sodium, or sodium stearyl fumarate.

The term "enol" used herein is not particularly limited, as long as it can be added to pharmaceutical preparations, but examples thereof include ascorbic acid or erythorbic acid and preferable examples include ascorbic acid.

The term "salt of the enol" used herein is not particularly limited, as long as it can be added to pharmaceutical preparations, but examples thereof include sodium ascorbate or sodium erythorbate and preferable examples include sodium ascorbate.

Fumaric acid is used in the orally disintegrating tablet according to the present invention and is solid at normal temperature.

The amount of fumaric acid contained in the orally disintegrating tablet according to the present invention is 0.1 to 15% by weight per 100% by weight of the orally disintegrating tablet.

A water-soluble polymer used in the present invention is hydroxypropyl cellulose.

The aforementioned hydroxypropyl cellulose is not limited as long as it maintains desired properties (disintegration time, hardness, dissolution properties) as an orally disintegrating tablet and examples of a commercially available product include the L grade (viscosity = 6 to 10 mPa*s) or the H grade (viscosity = 1000 to 4000 mPa*s) sold from Nippon Soda Co., Ltd.

The content of the water-soluble polymer in the tablet according to the present invention is 0.1 to 2.0% by weight, and particularly preferably 0.1 to 0.7% by weight per 100% by weight of the orally disintegrating tablet in view of formability and disintegrability and suspendability into water. When the content of the water-soluble polymer is too high, suspending takes too much time and the tablet becomes less suitable as an orally disintegrating tablet.

The orally disintegrating tablet according to the present invention may further comprise an excipient in addition to the aforementioned ingredients.

Examples of the excipient include organic excipients selected from sugars, sugar alcohols, starches, and celluloses; and inorganic excipients, whereas sugar alcohols and/or celluloses are preferred.

Examples of the sugars include one or combinations of two or more selected from lactose, sucrose, fructo-oligosaccharides, glucose, palatinose, maltose, reducing maltose, powdered sugar, powdered sucrose, fructose, isomerized lactose, and honey sugar.

Examples of the sugar alcohols include D-mannitol, erythritol, xylitol, maltitol, sorbitol, and the like and preferable examples are D-mannitol, erythritol, and xylitol and a further preferable example is D-mannitol. The D-mannitol may usually be any of those that are adapted to meet the Japanese Pharmacopoeia, the European Pharmacopoeia, and the United States Pharmacopoeia. The crystal form, the particle size, and the specific surface area of the D-mannitol to be incorporated are not particularly limited, but the crystal form may be any of the α type, the β type, the δ type, and amorphous; the particle size is preferably 10 µm or more and 250 µm or less and more preferably 20 µm or more and 150 µm or less; and the specific surface area is preferably 0.1 m²/g or more and 4 m²/g or less and more preferably 0.1 m²/g or more and 2 m²/g or less. The crystal form, the particle size, and the specific surface area can be measured by, for example, X-ray diffractometry, laser diffraction particle size distribution measurement, and measurement of BET specific surface area (multipoint), respectively. Examples of commercially available D-mannitol include D-mannitol from Merck & Co., Roquette, Towa Kasei Co., Ltd., Kao Corporation, and the like.

The incorporation amount of the aforementioned sugar alcohol can be selected as appropriate. When D-mannitol is used, the amount is usually 20 to 95% by weight and preferably 30 to 85% by weight relative to 100% by weight of the orally disintegrating tablet.

The aforementioned sugar alcohol may be incorporated in powder form with other ingredients and compression molded to prepare a tablet or may be granulated using an appropriate binder and then compression molded.

Examples of the celluloses include, in addition to microcrystalline cellulose, one or combinations of two or more selected from powdered cellulose, low-substituted hydroxypropyl cellulose, carmellose, carmellose calcium and croscarmellose sodium. The microcrystalline cellulose is usually a grade of microcrystalline cellulose having a bulk density of 0.10 to 0.46 g/cm³, preferably 0.10 to 0.42 g/cm³, and further preferably 0.10 to 0.26 g/cm³. Examples of commercially available microcrystalline cellulose include CEOLUS KG-1000 (bulk density 0.10 to 0.15 g/cm³), CEOLUS KG-802 (bulk density 0.13 to 0.23 g/cm³), and CEOLUS UF-711 (bulk density 0.20 to 0.26 g/cm³) (the foregoing are products made by Asahi Kasei Chemicals Corporation). Moreover, combinations of two or more kinds of microcrystalline cellulose different in bulk density adjusted to have a desired bulk density may be used.

The incorporation amount of the aforementioned microcrystalline cellulose is preferably 1 to 50% by weight per 100% by weight of the orally disintegrating tablet. If the amount exceeds 50% by weight, then the fluidity is deteriorated and the manufacturability may be decreased. The amount incorporated is more preferably 5 to 30% by weight.

The incorporation ratio of the aforementioned microcrystalline cellulose and sugar alcohol when the sugar alcohol is D-mannitol is 1 to 10 parts by weight, preferably 1.5 to 8.5 parts by weight, further preferably 2 to 3 parts by weight of sugar alcohol to 1 part of microcrystalline cellulose.

The aforementioned microcrystalline cellulose may be mixed in powder form with other ingredients and compression molded to prepare a tablet or may be granulated using an appropriate binder and then compression molded.

Examples of the starches include one or combinations of two or more selected from corn starch, potato starch, rice starch, and pregelatinized starch.

Examples of the inorganic excipients include one or combinations of two or more selected from synthetic hydrotalcite, precipitated calcium carbonate, hydrated silicon dioxide, light anhydrous silicic acid, magnesium aluminosilicate, and magnesium hydroxide.

The orally disintegrating tablet in the present invention further comprises, in addition to the aforementioned ingredients, a disintegrant.

The disintegrant is one or combinations of two or more selected from crospovidone (for example, products compatible with Japanese Pharmacopoeia), carmellose calcium (for example, products compatible with Japanese Pharmacopoeia), carmellose (for example, products compatible with Japanese Pharmacopoeia), croscarmellose sodium (for example, products compatible with Japanese Pharmacopoeia), corn starch (for example, products compatible with Japanese Pharmacopoeia), sodium starch glycolate (for example, products compatible with Japanese Pharmacopoeia), and pregelatinized starch; particularly preferable examples are one or combinations of two or more selected from crospovidone, carmellose calcium, carmellose, low-substituted hydroxypropyl cellulose, and pregelatinized starch; and a further preferable example is the combination of carmellose, crospovidone, and pregelatinized starch.

The aforementioned pregelatinized starch is starch pregelatinized by heat treatment and includes partly pregelatinized starch. Moreover, the aforementioned pregelatinized starch may be one described in Japanese Pharmaceutical Excipients. The average degree of gelatinization is preferably 90% or less and more preferably 70 to 80%. An example of commercially available pregelatinized starch is the pregelatinized starch swelstar PD-1 (manufactured by Asahi Kasei Chemicals Corporation).

The incorporation amount of the aforementioned pregelatinized starch is usually 1 to 15% by weight and preferably 1 to 10% by weight relative to 100% by weight of the orally disintegrating tablet.

The pregelatinized starch may be mixed in powder form with other ingredients and compression molded to prepare a tablet or may be granulated with other ingredients and then compression molded.

While the pregelatinized starch serves as the disintegrant in the orally disintegrating tablets according to the present invention, in production, it may also be sprayed onto powdered raw materials to promote granulation and granulate them, since it becomes viscous when dissolved or dispersed in a liquid such as water. Using this property, tablets having a good formability and desired intraoral disintegration can be obtained by spraying a solution or a liquid dispersion in which the pregelatinized starch is dissolved or dispersed in water onto a powder mixture containing microcrystalline cellulose with a bulk density of 0.26 g/cm³ or less and the sugar alcohol, producing granules by fluid-bed granulation, mixing other ingredients with this as needed, and compression molding. Such advantage in production is one of the characteristics unique to pregelatinized starch, which is hardly obtained when a conventional disintegrant such as low-substituted hydroxypropyl cellulose and crospovidone is used.

The incorporation amount of the aforementioned disintegrant is usually 0.5 to 20% by weight and preferably 2 to 20% by weight relative to 100% by weight of the orally disintegrating tablet.

The orally disintegrating tablet according to the present invention may comprise various additives usually used in production of tablets unless the effect of the invention is impeded.

Examples of the additives include a binder, a lubricant, a coating, a plasticizer, a colorant, a flavoring, an sweetener, a corrigent, a fluidizer, a foaming agent and a surfactant, and the like.

Examples of the binder include one or combinations of two or more selected from gum arabic, sodium alginate, carboxyvinyl polymer, gelatin, dextrin, pectin, sodium polyacrylate, pullulan, methyl cellulose, hydroxypropyl cellulose, hydroxypropyl methyl cellulose, polyvinyl alcohol, polyvinylpyrrolidone, and macrogol.

Examples of the lubricant include one or combinations of two or more selected from magnesium stearate (for example, products compatible swith Japanese Pharmacopoeia), calcium stearate (for example, products compatible with Japanese Pharmacopoeia), sodium stearyl fumarate (for example, products compatible with Japanese pharmaceutical excipients) and talc (for example, products compatible with Japanese Pharmacopoeia) and a particularly preferable example is magnesium stearate.

The incorporation amount of the lubricant is preferably 0.1 to 5.0% by weight per 100% by weight of the orally disintegrating tablet.

Examples of the coating that coats the surface of a powdered drug (the surface of crystals) or the surface of granules of a granulated drug include one or combinations of two or more selected from ethyl cellulose, aminoalkyl methacrylate copolymer E, methacrylic acid copolymer L, dried methacrylic acid copolymer LD, methacrylic acid copolymer LD, methacrylic acid copolymer S, aminoalkyl methacrylate copolymer RS, aminoalkyl methacrylate copolymer RS, ethyl acrylate-methyl methacrylate copolymer, polyvinyl acetal-diethylamino acetate, and polyvinyl acetate resin.

Examples of the plasticizer to be combined with the coating include one or combinations of two or more selected from diethyl sebacate, dibutyl sebacate, triethyl citrate, stearic acid, polyethylene glycol, and triacetin.

Examples of the colorant include one or combinations of two or more selected from food dyes such as Food Yellow No.5, Food Red No. 2, and Food Blue No.2; food lake dyes, Yellow Ferric Oxide, iron sesquioxide, titanium oxide, β-carotene, and riboflavin.

Examples of the flavoring include one or combinations of two or more selected from orange, lemon, strawberry, peppermint, menthol, Menthol Micron, and various flavors.

Examples of the sweetener include one or combinations of two or more selected from saccharin sodium, saccharin, aspartame, acesulfame potassium, dipotassium glycyrrhizinate, sucralose, stevia, and thaumatin.

Examples of the corrigent include one or combinations of two or more selected from sodium chloride, magnesium chloride, disodium inosinate, monosodium L-glutamate and honey.

Examples of the fluidizer include one or combinations of two or more selected from hydrated silicon dioxide, light anhydrous silicic acid, and talc.

Examples of the foaming agent include tartaric acid and/or anhydrous citric acid.

Examples of the surfactant include one or combinations of two or more selected from polyoxyl 40 stearate, sorbitan fatty acid esters, polyoxyethylene hydrogenated castor oils, polysorbate, glyceryl monostearate, and sodium lauryl sulfate.

The method for producing the orally disintegrating tablet according to the present invention may be a well-known method for producing solid formulations, and tablets can be obtained, for example, by granulating an active ingredient with additives, then adding a lubricant and the like, mixing them, and compressing them into tablets. Moreover, after the granulation, one or more operations such as drying and screening may be conducted as needed. Hereinafter, embodiments (Embodiments A and B) of the orally disintegrating tablet according to the present invention will be described together with the production method thereof.

Embodiment A: An orally disintegrating tablet obtained by compressing drug-free granules comprising a crystalline cellulose having a bulk density of 0.26 g/cm³ or less, a sugar alcohol and a pregelatinized starch (hereinafter, also referred to as rapidly disintegrating granules) and a drug or drug-containing granules.

In this Embodiment, the drug-free granules function as a skeleton of the formulation that can confer disintegrability and formability desirable in an orally disintegrating tablet. The drug-free granules exhibit excellent disintegrability and formability by just incorporating only the 3 ingredients of microcrystalline cellulose having a bulk density of 0.26 g/cm³ or less, a sugar alcohol, and pregelatinized starch, but other ingredients may be incorporated as needed. Moreover, the orally disintegrating tablet in this embodiment contains 0.1 to 2.0% by weight of hydroxypropyl cellulose as a water-soluble polymer relative to the total weight of the tablet and 0.1 to 15% by weight of fumaric acid relative to the total weight of the tablet, and therefore exhibits even more superior disintegrability and dissolution properties.

The method of production of the orally disintegrating tablet of Embodiment A comprises a step (A-1) of producing drug-free granules and a step (A-2) of producing drug-containing granules, and a step (A-3) of mixing the drug-free granules, a drug or drug-containing granules, and other ingredients, and compression molding the resulting mixture.

### A-1: Step of producing drug-free granules

The drug-free granules can be produced by the following method 1) or 2).

1) A process for wet-granulating a mixture containing microcrystalline cellulose with a bulk density of 0.26 g/cm³ or less, a sugar alcohol (for example, D-mannitol), and pregelatinized starch with water.
2) A process for granulating a mixture containing microcrystalline cellulose with a bulk density of 0.26 g/cm³ or less and a sugar alcohol (for example, D-mannitol) with a liquid such as water in which pregelatinized starch is dissolved or dispersed.

Here, the granulation may be granulation by conventional extrusion granulation, mixing agitation granulation, high-speed agitation granulation, fluid bed granulation, oscillating granulation, or the like.

The pregelatinized starch exhibits a viscosity suitable for granulation when dissolved or dispersed in a liquid, for example, water. Methods of granulation include a method involving mixing pregelatinized starch in powder form with other ingredients and granulating the resulting mixture with water, and a method of granulation with a liquid in which pregelatinized starch is dissolved or dispersed in water. Desired properties are conferred to tablets by either method, whereas the latter is preferred.

Moreover, when granulation is conducted with a liquid in which pregelatinized starch is dissolved or dispersed, either of high-speed agitation granulation and fluid bed granulation is applicable, whereas superior orally disintegrating tablets can be obtained when granules are produced by fluid bed granulation. When other ingredients such as a conventional disintegrant are to be mixed with drug-free granules, they may be incorporated into the mixtures before the granulation.

The incorporation ratio of the microcrystalline cellulose with a bulk density of 0.26 g/cm³ or less and sugar alcohol when the sugar alcohol is D-mannitol is 1.5 to 8.5 parts by weight or preferably 2 to 3 parts by weight of sugar alcohol to 1 part of microcrystalline cellulose.

### A-2: Step of producing drug-containing granules

The drug may be mixed with drug-free granules in powder form or after granulation as desired. The drug-containing granules may be produced by conventional extrusion granulation, mixing agitation granulation, high-speed agitation granulation, fluid bed granulation, or oscillating granulation.

The drug-containing granules may be produced, for example, by granulating a mixed powder of a powdered or granular drug (edoxaban tosylate hydrate), fumaric acid, microcrystalline cellulose having a bulk density of 0.26 g/cm³ or less, and a sugar alcohol with a liquid in which hydroxypropyl cellulose is dissolved or dispersed in water. Moreover, the drug-containing granules may be produced by granulating a mixed powder of a powdered or granular drug, fumaric acid, microcrystalline cellulose having a bulk density of 0.26 g/cm³ or less, a sugar alcohol, and hydroxypropyl cellulose with water.

The drug or the drug-containing granules may be coated for the masking of unpleasant taste such as bitter taste and irritancy and/or smells and/or the control of dissolution properties. For the coating, the aforementioned coating and a plasticizer may be used as appropriate. The method of coating involves, for example, use of a fluid bed granulation-coating machine, an agitation fluid bed granulation-coating machine, a centrifugal fluid granulation-coating machine, or a Wurster fluid bed granulation-coating machine.

When two or more drugs are to be used, the drugs may be incorporated into the same granules or incorporated into separate granules and processed by compression molding depending on the mixing compatibility between the drugs.

### A-3: Step of mixing drug-free granules, drug or drug-containing granules, and other ingredients and compression molding the resulting mixture

The drug-free granules and a drug or drug-containing granules, a disintegrant, and ingredients such as a a lubricant, and others, as desired, are mixed and processed by compression molding into orally disintegrating tablets. The mixing is conducted by using, for example, a tumbling mixer or a convection mixer. The drug may be mixed with other ingredients and used as a drug-containing mixed powder.

The compression molding of the orally disintegrating tablets according to the present invention may be conducted using a conventional tableting machine. The molding pressure of the tableting machine may be the same level as that for conventional tablets and is preferably about 2 to 20 kN and more preferably about 4 to 14 kN, while it depends on the shape and size of the tablet.

The incorporation ratio of the drug-free granules to the total weight of tablet ingredients may be 30 to 90%. When the drug is powdered, the incorporation ratio is 30 to 70% and preferably 30 to 60%. When the drug is used after granulation, the incorporation ratio is 30 to 70% and preferably 30 to 60%. Moreover, when the drug is used after granulation, the incorporation weight ratio of the drug-free granules to the drug-containing granules is preferably 0.5 to 2.0 of the drug-free granules to 1 of the drug-containing granules.

Embodiment B: An orally disintegrating tablet obtained by compression molding of a drug-free mixed powder containing microcrystalline cellulose having a bulk density of 0.26 g/cm³ or less, sugar alcohol, and pregelatinized starch and drug or drug-containing granules.

In this embodiment, the drug-free mixed powder provides desirable disintegration and formability in an orally disintegrating tablet. The drug-free granules exhibit excellent disintegrability and formability by just mixing only the 3 ingredients of microcrystalline cellulose having a bulk density of 0.26 g/cm³ or less, a sugar alcohol, and pregelatinized starch, but other ingredients may be mixed as needed. Moreover, the orally disintegrating tablet in this embodiment contains 0.1 to 2.0% by weight of a water-soluble polymer (hydroxypropyl cellulose) and an fumaric acid and therefore exhibits even more superior disintegrability and dissolution properties.

The method for producing the orally disintegrating tablets of Embodiment B comprises a step of producing drug-containing granules, as desired, and a step of mixing a drug or drug-containing granules and other ingredients and compression molding the resulting mixture. The step of producing drug-containing granules is the same as A-2 described above.

In the step of mixing a drug or drug-containing granules and other ingredients and compression molding the resulting mixture, the step of mixing or compression molding is the same as A-3 described above.

The orally disintegrating tablets according to the present invention thus obtained have excellent disintegrability and solubility when placed in the oral cavity or water and have excellent physical and chemical stability.

The disintegrability or the solubility of the orally disintegrating tablets according to the present invention is such that the disintegration and dissolution time of the tablet in the oral cavity (time until the tablet is completely disintegrated or dissolved in the oral cavity of a healthy adult man only with saliva without putting water in the mouth) is usually about 5 to 180 seconds, preferably about 5 to 60 seconds, and further preferably about 5 to 40 seconds.

The orally disintegrating tablets according to the present invention gradually disintegrate or dissolve with saliva when put in the mouth, but the tablets disintegrate or dissolve in a shorter time by intraoral pressure, that is, the pressure by the upper jaw and the tongue or the friction with the tongue, that is, the "licking" movement. For people with a dry mouth cavity or people with reduced saliva, the tablets may be disintegrated and/or dissolved with water or hot water in the oral cavity or may be taken with water as it is, in the same way as that for conventional tablets.

Meanwhile, the orally disintegrating tablets according to the present invention have sufficient hardness after a stability test under certain temperature and humidity conditions (for example, at a temperature of 25°C and a humidity of 75% in an open system for 1 week).

Therefore, the tablets have hardness sufficient to prevent disintegration in the production process and during distribution of the formulation, have practical hardness in storage under certain temperature and humidity conditions, and excellent storage stability.

The orally disintegrating tablets according to the present invention may be used for treating illness as formulations that are easy for elderly people, infants, and patients with dysphagia to take or as safe formulations for general adults.

### Examples

The following Examples are for illustrative purposes only and it is not intended to limit the present invention to these Examples.

### [Comparative Example 1-1]

A fluid bed granulator dryer (manufactured by Freund-Vector Corporation, model FLO-2) was charged with 384.9 g of edoxaban tosylate hydrate, 355.1 g of D-mannitol (manufactured by Roquette, Pearlitol 50C), 95.24 g of microcrystalline cellulose (manufactured by Asahi Kasei Chemicals Corporation, CEOLUS UF-711), 95.24 g of crospovidone (manufactured by BASF AG, Kollidon CL-F), and 57.14 g of carmellose (manufactured by Gotoku Chemical Company Ltd., NS-300), and the mixture was sprayed with a binding liquid in which 12.38 g of hydroxypropyl cellulose (manufactured by Nippon Soda Co., Ltd., HPC-H) was dissolved in 1364 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 451.4 g of rapidly disintegrating granules, 68.57 g of crospovidone (manufactured by BASF AG, Kollidon CL-F), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Example 1-1]

A fluid bed granulator dryer (manufactured by Freund-Vector Corporation, model FLO-2) was charged with 392.6 g of edoxaban tosylate hydrate, 384.6 g of D-mannitol (manufactured by Roquette, Pearlitol 50C), 95.24 g of microcrystalline cellulose (manufactured by Asahi Kasei Chemicals Corporation, CEOLUS UF-711), 5.714 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 7.619 g of hydroxypropyl cellulose (manufactured by Nippon Soda Co., Ltd., HPC-H) was dissolved in 839.0 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 437.7 g of rapidly disintegrating granules, 68.57 g of crospovidone (manufactured by BASF AG, Kollidon CL-F), 13.71 g of sucralose (manufactured by San-Ei Gen F.F.I., Inc.), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Example 1-2]

A fluid bed granulator dryer (manufactured by Freund-Vector Corporation, model FLO-2) was charged with 392.6 g of edoxaban tosylate hydrate, 371.2 g of D-mannitol (manufactured by Roquette, Pearlitol 50C), 95.24 g of microcrystalline cellulose (manufactured by Asahi Kasei Chemicals Corporation, CEOLUS UF-711), 19.05 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 7.619 g of hydroxypropyl cellulose (manufactured by Nippon Soda Co., Ltd., HPC-H) was dissolved in 839.0 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 437.7 g of rapidly disintegrating granules, 68.57 g of crospovidone (manufactured by BASF AG, Kollidon CL-F), 13.71 g of sucralose (manufactured by San-Ei Gen F.F.I., Inc.), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Example 1-3]

A fluid bed granulator dryer (manufactured by Freund-Vector Corporation, Model FLO-2) was charged with 384.9 g of edoxaban tosylate hydrate, 171.3 g of D-mannitol (manufactured by Roquette, Pearlitol 50C), 95.24 g of microcrystalline cellulose (manufactured by Asahi Kasei Chemicals Corporation, CEOLUS UF-711), 228.6 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 5.714 g of hydroxypropyl cellulose (manufactured by Nippon Soda Co., Ltd., HPC-H) was dissolved in 629.2 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 451.4 g of rapidly disintegrating granules, 68.57 g of crospovidone (manufactured by BASF AG, Kollidon CL-F), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Comparative Example 2-1]

A fluid bed granulator dryer (manufactured by Freund-Vector Corporation, Model FLO-2) was charged with 384.9 g of edoxaban tosylate hydrate, 355.1 g of D-mannitol (manufactured by Roquette, Pearlitol 50C), 95.24 g of microcrystalline cellulose (manufactured by Asahi Kasei Chemicals Corporation, CEOLUS UF-711), 95.24 g of crospovidone (manufactured by BASF AG, Kollidon CL-F), and 57.14 g of carmellose (manufactured by Gotoku Chemical Company Ltd., NS-300), and the mixture was sprayed with a binding liquid in which 12.38 g of hydroxypropyl cellulose (manufactured by Nippon Soda Co., Ltd., HPC-H) was dissolved in 1364 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 451.4 g of rapidly disintegrating granules, 68.57 g of crospovidone (manufactured by BASF AG, Kollidon CL-F), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Comparative Example 2-2]

A fluid bed granulator dryer (manufactured by Freund-Vector Corporation, model FLO-2) was charged with 384.9 g of edoxaban tosylate hydrate, 352.3 g of D-mannitol (manufactured by Roquette, Pearlitol 50C), 95.24 g of microcrystalline cellulose (manufactured by Asahi Kasei Chemicals Corporation, CEOLUS UF-711), 5.714 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 47.62 g of hydroxypropyl cellulose (manufactured by Nippon Soda Co., Ltd., HPC-H) was dissolved in 632.9 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 330.0 g of the obtained drug-containing granules, 248.3 g of rapidly disintegrating granules, 37.71 g of crospovidone (manufactured by BASF AG, Kollidon CL-F), and 12.60 g of magnesium stearate (manufactured by Mallinckrodt Pharmaceuticals, HyQual 5712) were mixed and then the mixture was tableted with a rotary tableting machine (Kikusui Seisakusho Ltd., 18HUK) at a compression force of 5 kN to obtain orally disintegrating tablets (11 mm ϕ, 400 mg).

### [Comparative Example 2-3]

A fluid bed granulator dryer (manufactured by Freund-Vector Corporation, model FLO-2) was charged with 384.9 g of edoxaban tosylate hydrate, 352.3 g of D-mannitol (manufactured by Roquette, Pearlitol 50C), 95.24 g of microcrystalline cellulose (manufactured by Asahi Kasei Chemicals Corporation, CEOLUS UF-711), 5.714 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 47.62 g of hydroxypropyl cellulose (manufactured by Nippon Soda Co., Ltd., HPC-H) was dissolved in 3920 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 330.0 g of the obtained drug-containing granules, 248.3 g of rapidly disintegrating granules, 37.71 g of crospovidone (manufactured by BASF AG, Kollidon CL-F), and 12.60 g of magnesium stearate (manufactured by Mallinckrodt Pharmaceuticals, HyQual 5712) were mixed and then the mixture was tableted with a rotary tableting machine (Kikusui Seisakusho Ltd., 18HUK) at a compression force of 5 kN to obtain orally disintegrating tablets (11 mm ϕ, 400 mg).

### [Example 2-1]

A fluid bed granulator dryer (manufactured by Freund-Vector Corporation, model FLO-2) was charged with 384.9 g of edoxaban tosylate hydrate, 392.3 g of D-mannitol (manufactured by Roquette, Pearlitol 50C), 95.24 g of microcrystalline cellulose (manufactured by Asahi Kasei Chemicals Corporation, CEOLUS UF-711), 5.714 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 7.619 g of hydroxypropyl cellulose (manufactured by Nippon Soda Co., Ltd., HPC-H) was dissolved in 101.3 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 330.0 g of the obtained drug-containing granules, 248.3 g of rapidly disintegrating granules, 37.71 g of crospovidone (manufactured by BASF AG, Kollidon CL-F), and 12.60 g of magnesium stearate (manufactured by Mallinckrodt Pharmaceuticals, HyQual 5712) were mixed and then the mixture was tableted with a rotary tableting machine (Kikusui Seisakusho Ltd., 18HUK) at a compression force of 5 kN to obtain orally disintegrating tablets (11 mm ϕ, 400 mg).

### [Example 2-2]

A fluid bed granulator dryer (manufactured by Freund-Vector Corporation, model FLO-2) was charged with 392.6 g of edoxaban tosylate hydrate, 384.6 g of D-mannitol (manufactured by Roquette, Pearlitol 50C), 95.24 g of microcrystalline cellulose (manufactured by Asahi Kasei Chemicals Corporation, CEOLUS UF-711), 5.714 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 7.619 g of hydroxypropyl cellulose (manufactured by Nippon Soda Co., Ltd., HPC-H) was dissolved in 839.0 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 437.7 g of rapidly disintegrating granules, 68.57 g of crospovidone (manufactured by BASF AG, Kollidon CL-F), 13.71 g of sucralose (manufactured by San-Ei Gen F.F.I., Inc.), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Comparative Example 3-1]

16.16 g of edoxaban tosylate hydrate, 15.92 g of D-mannitol (manufactured by Roquette, Pearlitol 50C), and 4.0 g of microcrystalline cellulose (manufactured by Asahi Kasei Chemicals Corporation, CEOLUS UF-711) was mixed using a mortar to obtain a mixture (drug-containing mixed powder). 36.08 g of the obtained drug-containing mixed powder and 0.72 g of magnesium stearate (manufactured by Mallinckrodt Pharmaceuticals, HyQual 5712) were mixed and 0.1 g of fumaric acid (manufactured by Nippon Shokubai) and 0.1 g of hydroxypropyl cellulose (manufactured by Nippon Soda Co., Ltd., HPC-L) were further admixed, and then the mixture was tableted with a bench-top tablet molding machine (Ichihashi Seiki, HANDTAB) at a compression force of 10 kN to obtain orally disintegrating tablets (8.0 mm ϕ, 194 mg).

### [Comparative Example 3-2]

16.16 g of edoxaban tosylate hydrate, 15.92 g of D-mannitol (manufactured by Roquette, Pearlitol 50C), and 4.0 g of microcrystalline cellulose (manufactured by Asahi Kasei Chemicals Corporation, CEOLUS UF-711) was mixed using a mortar to obtain a mixture (drug-containing mixed powder). 36.08 g of the obtained drug-containing mixed powder and 0.72 g of magnesium stearate (manufactured by Mallinckrodt Pharmaceuticals, HyQual 5712) were mixed and 0.1 g of crospovidone (manufactured by BASF AG, Kollidon CL-F) and 0.1 g of carmellose (manufactured by Gotoku Chemical Company Ltd., NS-300) were further admixed, and then the mixture was tableted with a bench-top tablet molding machine (Ichihashi Seiki, HANDTAB) at a compression force of 10 kN to obtain orally disintegrating tablets (8.0 mm ϕ, 194 mg).

### [Example 3-1]

A fluid bed granulator dryer (manufactured by Freund-Vector Corporation, model FLO-2) was charged with 392.6 g of edoxaban tosylate hydrate, 371.2 g of D-mannitol (manufactured by Roquette, Pearlitol 50C), 95.24 g of microcrystalline cellulose (manufactured by Asahi Kasei Chemicals Corporation, CEOLUS UF-711), 19.05 g of fumaric acid (manufactured by Merck), 57.14 g of crospovidone (manufactured by BASF AG, Kollidon CL-F), and 57.14 g of carmellose (manufactured by Gotoku Chemical Company Ltd., NS-300), and the mixture was sprayed with a binding liquid in which 7.619 g of hydroxypropyl cellulose (manufactured by Nippon Soda Co., Ltd., HPC-H) was dissolved in 839.0 g of purified water and then dried to obtain granules (drug-containing granules). Moreover, a fluid bed granulator dryer (manufactured by Powrex Corporation, GPCG-15) was charged with 18480 g of D-mannitol (manufactured by Roquette, Pearlitol 50C) and 8360 g of microcrystalline cellulose (manufactured by Asahi Kasei Chemicals Corporation, CEOLUS KG-1000), and the mixture was sprayed with a liquid in which 1100 g of pregelatinized starch (manufactured by Asahi Kasei Chemicals Corporation, swelstar PD-1) was dispersed in 12650 g of purified water and then dried to obtain granules (rapidly disintegrating granules). Furthermore, 600.0 g of the obtained drug-containing granules, 437.7 g of rapidly disintegrating granules, 68.57 g of crospovidone (manufactured by BASF AG, Kollidon CL-F), 13.71 g of sucralose (manufactured by San-Ei Gen F.F.I., Inc.), and 22.86 g of magnesium stearate (manufactured by Mallinckrodt Pharmaceuticals, HyQual 5712) were mixed and then the mixture was tableted with a rotary tableting machine (Kikusui Seisakusho Ltd., VIRGO) at a compression force of 5 kN to obtain orally disintegrating tablets (11 mm ϕ, 400 mg).

### [Method of evaluation]

The tablets obtained in the Examples and Comparative Examples were evaluated by the following method.

The tablet thickness and the hardness were measured using a fully automated tablet testing instrument (Type WHT-2, PHARMA TEST APPARATEBAU GmbH) (average of 20 tablets is described).

The disintegration test was conducted referring to "Disintegration Test" in the Japanese Pharmacopoeia 16th edition (the maximum value of 6 tablets is described).

The dissolution test was conducted according to the second method (the paddle method, 50 rpm) described in the Japanese Pharmacopoeia. The percentage dissolution was expressed as a mean percentage dissolution of 6 tablets. The dissolution test solution (pH 6.0) used was a solution obtained by adding an appropriate quantity of a citric acid solution (0.025 mol/L) to a disodium hydrogen phosphate solution (0.05 mol/L) and adjusting the solution to pH 6.0.

The results are shown in Table 1 to Table 5.

### [Brief description of results]

Table 1: In comparison with formulations in which no fumaric acid is incorporated, such as Comparative Example 1-1, tablets in which fumaric acid is incorporated, such as Examples 1-1 to 1-3, disintegrate rapidly within 60 seconds and exhibit superior functionality as orally disintegrating tablets. Moreover, an increase in the incorporation amount of fumaric acid assures more rapid disintegration and dissolution.

Table 2: In comparison with formulations in which no fumaric acid is incorporated, such as Comparative Example 2-1, or formulations with a large amount of hydroxypropyl cellulose incorporated, such as Comparative Examples 2-2 and 2-3, formulations in which fumaric acid is incorporated and a small amount of hydroxypropyl cellulose is incorporated, such as Examples 2-1 and 2-2, exhibit a short disintegration time of 60 seconds or less and superior functionality as orally disintegrating tablets. Furthermore, the disintegration time in this case is short, regardless of the physical properties of the hydroxypropyl cellulose.

Table 3: In comparison with formulations in which fumaric acid and hydroxypropyl cellulose are incorporated, but no disintegrant is incorporated, such as Comparative Example 3-1, or formulations in which a disintegrant is incorporated, but neither fumaric acid nor hydroxypropyl cellulose is incorporated, such as Comparative Example 3-2, tablets in which a disintegrant, fumaric acid, and a small amount of hydroxypropyl cellulose are incorporated, such as Example 3-1, exhibit a short disintegration time of 60 seconds or less and superior functionality as orally disintegrating tablets.

Table 4 and Table 5: Orally disintegrating tablets of Example 4 were produced according to the method of production described in Examples 1 to 3 and the formulation set forth in Table 4. Tablets in which fumaric acid, and a small amount of hydroxypropyl cellulose, and a disintegrant are incorporated with edoxaban, such as Examples 4-1 to 4-9, exhibit a short disintegration time of 60 seconds or less and superior functionality as orally disintegrating tablets, as well as rapid dissolution.

Table 6 and Table 7: Illustrate examples of the formulation according to the present invention.

**[Table 1]**

| Comparative Example/Example | Comparative Example 1-1 | Example 1-1 | Example 1-2 | Example 1-3 |
|---|---|---|---|---|
| Incorporation amount of fumaric acid (%) | 0 | 0.3 | 1 | 12 |
| Incorporation amount of hydroxypropyl cellulose (%) | 0.65 | 0.4 | 0.4 | 0.3 |
| Viscosity of hydroxypropyl cellulose (mPa*s) (20°C, 2% aqueous solution) | 2790 | 2790 | 2790 | 2790 |
| Molecular weight of hydroxypropyl cellulose* | -910,000- | -910,000- | -910,000- | -910,000- |
| Tablet thickness (average, mm) | 5.7 | 5.6 | 5.6 | 5.5 |
| Tablet hardness (average, kg) | 3.4 | 3.4 | 3.4 | 3.3 |
| Disintegration time (maximum of individuals, sec) | 84 | 36 | 28 | 26 |
| Percentage dissolution at Time 15 min. (pH 6.0, %) | 71 | 82 | 90 | 95 |
| Percentage dissolution at Time 30 min. (pH 6.0, %) | 82 | 90 | 96 | 98 |
| Percentage dissolution at Time 45 min. (pH 6.0, %) | 87 | 94 | 98 | 99 |

| | | | | |
|---|---|---|---|---|
| * Values on manufacturer's catalog | | | | |

**[Table 2]**

| Comparative Example/Example | Comparative Example 2-1 | Comparative Example 2-2 | Comparative Example 2-3 | Example 2-1 | Example 2-2 |
|---|---|---|---|---|---|
| Incorporation amount of fumaric acid (%) | 0 | 0.3 | 0.3 | 0.3 | 0.3 |
| Viscosity of hydroxypropyl cellulose (mPa*s) (20°C, 2% aqueous solution) | 2790 | 8.3 | 2790 | 8.3 | 2790 |
| Molecular weight of hydroxypropyl cellulose* | -910,000- | -140,000- | -910,000- | -140,000- | -910,000- |
| Incorporation amount of HPC (%) | 0.65 | 2.5 | 2.5 | 0.4 | 0.4 |
| Uncoated tablet thickness (average, mm) | 5.7 | 5.6 | 5.6 | 5.7 | 5.6 |
| Uncoated tablet hardness (average, kg) | 3.4 | 5.2 | 5.1 | 3.5 | 3.4 |
| Disintegration time (maximum of individuals, sec) | 84 | 64 | 75 | 34 | 36 |
| Percentage dissolution at Time 15 min. (pH 6.0, %) | 71 | 90 | 82 | 84 | 82 |
| Percentage dissolution at Time 30 min. (pH 6.0, %) | 82 | 94 | 92 | 90 | 90 |
| Percentage dissolution at Time 45 min. (pH 6.0, %) | 87 | 96 | 95 | 92 | 94 |

| | | | | | |
|---|---|---|---|---|---|
| * Values on manufacturer's catalog | | | | | |

**[Table 3]**

| Comparative Example/Example | | Comparative Example 3-1 | Comparative Example 3-2 | Example 3-1 |
|---|---|---|---|---|
| Presence or absence of incorporated additives | Fumaric acid | present | absent | present |
| | Carmellose | absent | present | present |
| | Crospovidone | absent | present | present |
| | Hydroxypropyl cellulose | present | absent | present |
| Disintegration time (maximum of individuals, sec) | | > 600 | 61 | 28 |

**[Table 4]**

| Comparative Example/Example | | Example 4-1 | Example 4-2 | Example 4-3 | Example 4-4 | Example 4-5 | Example 4-6 |
|---|---|---|---|---|---|---|---|
| Edoxaban tosylate hydrate (in terms of edoxaban) | | 80.8 (60) | 80.8 (60) | 80.8 (60) | 20.2 (15) | 40.4 (30) | 80.8 (60) |
| Ingredients (mg) | D-mannitol | 185.28 | 182.48 | 158.48 | 39.528 | 79.14 | 158.09 |
| | Microcrystalline cellulose | 66.6 | 66.6 | 66.6 | 13.9 | 27.8 | 55.5 |
| | Crospovidone | 36 | 36 | 36 | 7.7 | 15.5 | 30.9 |
| | Carmellose | 12 | 12 | 12 | 3 | 6 | 12 |
| | Pregelatinized starch | 6.1 | 6.1 | 6.1 | 1.2 | 2.3 | 4.7 |
| | Hydroxypropyl cellulose | 1.6 | 1.6 | 1.6 | 0.4 | 0.8 | 1.6 |
| | Fumaric acid | 1.2 | 4 | 28 | 1 | 2 | 4 |
| | Saccharin sodium hydrate | 2 | 2 | 2 | 1.2 | 2.4 | 4.8 |
| | Magnesium stearate | 8 | 8 | 8 | 1.8 | 3.6 | 7.2 |
| | Yellow Ferric Oxide | 0.36 | 0.36 | 0.36 | 0.09 | - | 0.36 |
| | Iron sesquioxide | 0.04 | 0.04 | 0.04 | - | 0.036 | - |
| Uncoated tablet mass (mg) | | 400 | 400 | 400 | 90 | 180 | 360 |
| Uncoated tablet thickness (average, mm) | | 5.2 | 5.2 | 5.2 | 3.4 | 3.8 | 4.5 |
| Uncoated tablet hardness (average, kg) | | 6.2 | 6.2 | 5.9 | 3.1 | 5.1 | 5.5 |
| Disintegration time (maximum of individuals, sec) | | 29 | 29 | 28 | 23 | 33 | 36 |
| Percentage dissolution at Time 15 min. (pH 6.0, %) | 84 | 93 | 99 | 97 | 95 | 92 | |
| Percentage dissolution at Time 30 min. (pH 6.0, %) | 92 | 98 | 101 | 100 | 99 | 98 | |
| Percentage dissolution at Time 45 min. (pH 6.0, %) | 95 | 99 | 101 | 101 | 99 | 100 | |

**[Table 5]**

| Comparative Example/Example | | Example 4-7 | Example 4-8 | Example 4-9 |
|---|---|---|---|---|
| Edoxaban tosylate hydrate (in terms of edoxaban) | | 20.2 (15) | 40.4 (30) | 80.8 (60) |
| Ingredients (mg) | D-mannitol | 45.003 | 90.164 | 180.116 |
| | Microcrystalline cellulose | 16.358 | 32.788 | 65.48 |
| | Crospovidone | 9 | 18 | 36 |
| | Carmellose | 3 | 6 | 12 |
| | Pregelatinized starch | 1.494 | 2.998 | 5.984 |
| | Hydroxypropyl cellulose | 0.4 | 0.8 | 1.6 |
| | Fumaric acid | 1 | 2 | 4 |
| | Saccharin sodium hydrate | 1.3 | 2.6 | 5.2 |
| | Magnesium stearate | 2 | 4 | 8 |
| | Yellow Ferric Oxide | 0.1 | - | 0.4 |
| | Iron sesquioxide | 0.04 | 0.04 | - |
| | Orange flavor | 0.1 | 0.2 | 0.4 |
| Uncoated tablet mass (mg) | | 100 | 200 | 400 |
| Uncoated tablet thickness (average, mm) | | 3.4 | 4.2 | 4.4 |
| Uncoated tablet hardness (average, kg) | | 4.0 | 4.8 | 7.1 |
| Disintegration time (maximum of individuals, sec) | | 22 | 21 | 32 |
| Percentage dissolution at Time 15 min. (pH 6.0, %) | | 100 | 97 | 94 |
| Percentage dissolution at Time 30 min. (pH 6.0, %) | | 104 | 102 | 99 |
| Percentage dissolution at Time 45 min. (pH 6.0, %) | | 104 | 103 | 101 |

**[Table 6]**

| Formulation | | 1 | 2 | 3 | 4 | 5 |
|---|---|---|---|---|---|---|
| Edoxaban tosylate hydrate | | 80.8 | 20.2 | 80.8 | 20.2 | 80.8 |
| (in terms of edoxaban) | | 60 | 15 | 60 | 15 | 60 |
| Ingredients (mg) | D-mannitol | 185.216 | 32.748 | 182.436 | 31.756 | 202.016 |
| | Microcrystalline cellulose | 40 | 16.358 | 52.28 | 19 | 50 |
| | Crospovidone | 40 | 5 | 20 | 6 | 25 |
| | Carmellose | 16 | 5 | 30 | 7 | 10 |
| | Pregelatinized starch | 5.984 | 1.494 | 5.984 | 1.494 | 5.984 |
| | Hydroxypropyl cellulose | 0.4 | 1 | 2 | 0.65 | 6 |
| | Fumaric acid | - | 13 | 18 | - | - |
| | Alginic acid | - | - | - | 10 | 2 |
| | Aspartic acid | 10 | - | - | - | 2 |
| | Saccharin sodium hydrate | 5.2 | 1 | - | 0.5 | 7 |
| | Magnesium stearate | - | - | 8 | - | - |
| | Sodium stearyl fumarate | 15 | 4 | - | 3 | 8 |
| | Yellow Ferric Oxide | 0.4 | 0.1 | 0.5 | 0.1 | 1 |
| | Iron sesquioxide | - | - | - | 0.1 | - |
| | Orange flavor | 1 | 0.1 | - | 0.2 | 0.2 |
| Uncoated tablet mass (mg) | | 400 | 100 | 400 | 100 | 400 |

**[Table 7]**

| Formulation | | 6 | 7 | 8 | 9 |
|---|---|---|---|---|---|
| Edoxaban tosylate hydrate | | 40.4 | 40.4 | 20.2 | 40.4 |
| (in terms of edoxaban) | | 30 | 30 | 15 | 30 |
| Ingredients (mg) | D-mannitol | 92.414 | 101.66 | 42.21 | 94 |
| | Microcrystalline cellulose | 31.788 | 35 | 13 | 30 |
| | Crospovidone | 12 | 6 | 11 | 18 |
| | Carmellose | 6 | 4 | 4 | 6 |
| | Pregelatinized starch | 2.998 | 2.998 | 1.494 | 2.998 |
| | Hydroxypropyl cellulose | 0.8 | 0.3 | 0.7 | 0.8 |
| | Fumaric acid | - | - | - | 1 |
| | Alginic acid | 3 | 4 | - | - |
| | Aspartic acid | 3 | - | 5 | 3 |
| | Saccharin sodium hydrate | 2.6 | 0.5 | 1.3 | 0.5 |
| | Magnesium stearate | 5 | - | 1 | - |
| | Sodium stearyl fumarate | - | 5 | - | 3 |
| | Yellow Ferric Oxide | - | - | - | 0.2 |
| | Iron sesquioxide | - | 0.04 | 0.1 | - |
| | Orange flavor | - | 0.1 | - | 0.1 |
| Uncoated tablet mass (mg) | | 200 | 200 | 100 | 100 |

## Claims

1. An orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (D) one or more disintegrant selected from the group consisting of carmellose, crospovidone, carmellose calcium, croscarmellose sodium, corn starch, sodium starch glycolate, and pregelatinized starch.

2. The orally disintegrating tablet according to claim 1, wherein the disintegrant is carmellose, crospovidone, and pregelatinized starch.

3. The orally disintegrating tablet according to claim 1 or 2, further comprising a sugar alcohol and microcrystalline cellulose having a bulk density of 0.26 g/cm³ or less.

4. The orally disintegrating tablet according to claim 3, wherein the sugar alcohol is D-mannitol.

5. An orally disintegrating tablet comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, (E) carmellose, (F) D-mannitol, (G) microcrystalline cellulose having a bulk density of 0.26 g/cm³ or less, and (H) pregelatinized starch.

6. The orally disintegrating tablet according to claim 5, comprising: a drug-containing mixed powder or drug-containing granules comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose; and a drug-free mixed powder comprising (F) D-mannitol, (G) microcrystalline cellulose having a bulk density of 0.26 g/cm³ or less, and (H) pregelatinized starch.

7. The orally disintegrating tablet according to claim 5, comprising: a drug-containing mixed powder or drug-containing granules comprising (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose; and drug-free granules comprising (F) D-mannitol, (G) microcrystalline cellulose having a bulk density of 0.26 g/cm³ or less, and (H) pregelatinized starch.

8. The orally disintegrating tablet according to any one of claims 5 to 7, further comprising one or more ingredients selected from the group consisting of crospovidone, carmellose calcium, croscarmellose sodium, corn starch and sodium starch glycolate.

9. The orally disintegrating tablet according to any one of claims 5 to 7, further comprising crospovidone.

10. The orally disintegrating tablet according to any one of claims 5 to 9, wherein the tablet disintegrates within 60 seconds in the "Disintegration Test" in the Japanese Pharmacopoeia 16^{th} edition, wherein the result is the maximum value of 6 tablets.

11. A method for producing an orally disintegrating tablet, comprising:
a step of producing drug-containing granules by either:
mixing (A) edoxaban tosylate hydrate, (B) 0.1 to 15% by weight of fumaric acid relative to the total weight of the tablet, and (E) carmellose, and spraying, onto the mixture, (C) 0.1 to 2.0% by weight of hydroxypropyl cellulose relative to the total weight of the tablet, dissolved in water; or
mixing (A) edoxaban tosylate hydrate, (B) 0.1 to 15% by weight of fumaric acid relative to the total weight of the tablet, (C) 0.1 to 2.0% by weight of hydroxypropyl cellulose relative to the total weight of the tablet, and (E) carmellose, and spraying water onto the mixture;
a step of producing drug-free granules by mixing (F) D-mannitol and (G) microcrystalline cellulose having a bulk density of 0.26 g/cm³ or less, and spraying, onto the mixture, (H) pregelatinized starch dissolved or dispersed in water to produce drug-free granules; and
a step of compression molding the obtained two types of granules.

## Patentansprüche

1. Oral zerfallende Tablette, umfassend (A) Edoxabantosylat-Hydrat, (B) 0,1 bis 15 Gew.-% Fumarsäure, bezogen auf das Gesamtgewicht der Tablette, (C) 0,1 bis 2,0 Gew.-% Hydroxypropylcellulose, bezogen auf das Gesamtgewicht der Tablette, und (D) ein oder mehrere Zerfallsmittel, die aus der Gruppe bestehend aus Carmellose, Crospovidon, Carmellose-Calcium, Croscarmellose-Natrium, Maisstärke, Natriumstärkeglykolat und vorgelierter Stärke ausgewählt ist.

2. Oral zerfallende Tablette nach Anspruch 1, wobei das Zerfallsmittel Carmellose, Crospovidon und vorgelierte Stärke ist.

3. Oral zerfallende Tablette nach Anspruch 1 oder 2, ferner umfassend einen Zuckeralkohol und mikrokristalline Cellulose mit einer Schüttdichte von 0,26 g/cm³ oder weniger.

4. Oral zerfallende Tablette nach Anspruch 3, wobei der Zuckeralkohol D-Mannit ist.

5. Oral zerfallende Tablette, umfassend (A) Edoxabantosylat-Hydrat, (B) 0,1 bis 15 Gew.-% Fumarsäure, bezogen auf das Gesamtgewicht der Tablette, (C) 0,1 bis 2,0 Gew.-% Hydroxypropylcellulose, bezogen auf das Gesamtgewicht der Tablette, (E) Carmellose, (F) D-Mannit, (G) mikrokristalline Cellulose mit einer Schüttdichte von 0,26 g/cm³ oder weniger und (H) vorgelierte Stärke.

6. Oral zerfallende Tablette nach Anspruch 5, umfassend: ein wirkstoffhaltiges gemischtes Pulver oder ein wirkstoffhaltiges Granulat, umfassend (A) Edoxabantosylat-Hydrat, (B) 0,1 bis 15 Gew.-% Fumarsäure, bezogen auf das Gesamtgewicht der Tablette, (C) 0,1 bis 2,0 Gew.-% Hydroxypropylcellulose, bezogen auf das Gesamtgewicht der Tablette, und (E) Carmellose, und ein wirkstofffreies gemischtes Pulver, umfassend (F) D-Mannit, (G) mikrokristalline Cellulose mit einer Schüttdichte von 0,26 g/cm³ oder weniger und (H) vorgelierte Stärke.

7. Oral zerfallende Tablette nach Anspruch 5, umfassend: ein wirkstoffhaltiges gemischtes Pulver oder ein wirkstoffhaltiges Granulat, umfassend (A) Edoxabantosylat-Hydrat, (B) 0,1 bis 15 Gew.-% Fumarsäure, bezogen auf das Gesamtgewicht der Tablette, (C) 0,1 bis 2,0 Gew.-% Hydroxypropylcellulose, bezogen auf das Gesamtgewicht der Tablette, und (E) Carmellose, und ein wirkstofffreies Granulat, umfassend (F) D-Mannit, (G) mikrokristalline Cellulose mit einer Schüttdichte von 0,26 g/cm³ oder weniger und (H) vorgelierte Stärke.

8. Oral zerfallende Tablette nach einem der Ansprüche 5 bis 7, ferner umfassend einen oder mehrere Bestandteile, die aus der Gruppe bestehend aus Crospovidon, Carmellose-Calcium, Croscarmellose-Natrium, Maisstärke und Natriumstärkeglykolat ausgewählt sind.

9. Oral zerfallende Tablette nach einem der Ansprüche 5 bis 7, ferner umfassend Crospovidon.

10. Oral zerfallende Tablette nach einem der Ansprüche 5 bis 9, wobei die Tablette im "Disintegration Test" in der japanischen Pharmakopöe, 16. Ausgabe, innerhalb von 60 Sekunden zerfällt, wobei das Ergebnis der Höchstwert von 6 Tabletten ist.

11. Verfahren zur Produktion einer oral zerfallenden Tablette, umfassend:
einen Schritt des Produzierens von wirkstoffhaltigem Granulat durch entweder:
Mischen von (A) Edoxabantosylat-Hydrat, (B) 0,1 bis 15 Gew.-% Fumarsäure, bezogen auf das Gesamtgewicht der Tablette, und (E) Carmellose, und Sprühen von (C) 0,1 bis 2,0 Gew.-% Hydroxypropylcellulose, bezogen auf das Gesamtgewicht der Tablette, in Wasser gelöst, auf das Gemisch; oder
Mischen von (A) Edoxabantosylat-Hydrat, (B) 0,1 bis 15 Gew.-% Fumarsäure, bezogen auf das Gesamtgewicht der Tablette, (C) 0,1 bis 2,0 Gew.-% Hydroxypropylcellulose, bezogen auf das Gesamtgewicht der Tablette, und (E) Carmellose, und Sprühen von Wasser auf das Gemisch;
einen Schritt des Produzierens von wirkstofffreiem Granulat durch Mischen von (F) D-Mannit und (G) mikrokristalliner Cellulose mit einer Schüttdichte von 0,26 g/cm³ oder weniger, und Sprühen von (H) vorgelierter Stärke, in Wasser gelöst oder dispergiert, auf das Gemisch, um wirkstofffreies Granulat zu produzieren; und
einen Schritt eines Formpressens der erhaltenen zwei Granulattypen.

## Revendications

1. Comprimé orodispersible comprenant (A) de l'hydrate de tosylate d'edoxaban, (B) de 0,1 à 15 % en poids d'acide fumarique par rapport au poids total du comprimé, (C) de 0,1 à 2,0 % en poids d'hydroxypropylcellulose par rapport au poids total du comprimé et (D) un ou plusieurs désintégrants choisis dans le groupe constitué de : carmellose, crospovidone, carmellose calcique, croscarmellose sodique, amidon de maïs, glycolate d'amidon de sodium et amidon prégélatinisé.

2. Comprimé orodispersible selon la revendication 1, dans lequel le désintégrant est : carmellose, crospovidone et amidon prégélatinisé.

3. Comprimé orodispersible selon la revendication 1 ou 2, comprenant en outre un alcool de sucre et une cellulose microcristalline ayant une masse volumique en vrac de 0,26 g/cm³ ou moins.

4. Comprimé orodispersible selon la revendication 3, dans lequel l'alcool de sucre est le D-mannitol.

5. Comprimé orodispersible comprenant (A) de l'hydrate de tosylate d'edoxaban, (B) de 0,1 à 15 % en poids d'acide fumarique par rapport au poids total du comprimé, (C) de 0,1 à 2,0 % en poids d'hydroxypropylcellulose par rapport au poids total du comprimé, (E) du carmellose, (F) du D-mannitol, (G) une cellulose microcristalline ayant une masse volumique en vrac de 0,26 g/cm³ ou moins et (H) de l'amidon prégélatinisé.

6. Comprimé orodispersible selon la revendication 5, comprenant : une poudre mélangée contenant un médicament ou des granulés contenant un médicament comprenant (A) de l'hydrate de tosylate d'edoxaban, (B) de 0,1 à 15 % en poids d'acide fumarique par rapport au poids total du comprimé, (C) de 0,1 à 2,0 % en poids d'hydroxypropylcellulose par rapport au poids total du comprimé et (E) du carmellose ; et une poudre mélangée sans médicament comprenant (F) du D-mannitol, (G) une cellulose microcristalline ayant une masse volumique en vrac de 0,26 g/cm³ ou moins et (H) de l'amidon prégélatinisé.

7. Comprimé orodispersible selon la revendication 5, comprenant : une poudre mélangée contenant un médicament ou des granulés contenant un médicament comprenant (A) de l'hydrate de tosylate d'edoxaban, (B) de 0,1 à 15 % en poids d'acide fumarique par rapport au poids total du comprimé, (C) de 0,1 à 2,0 % en poids d'hydroxypropylcellulose par rapport au poids total du comprimé et (E) du carmellose ; et des granulés sans médicament comprenant (F) du D-mannitol, (G) une cellulose microcristalline ayant une masse volumique en vrac de 0,26 g/cm³ ou moins et (H) de l'amidon prégélatinisé.

8. Comprimé orodispersible selon l'une quelconque des revendications 5 à 7, comprenant en outre un ou plusieurs ingrédients choisis dans le groupe constitué de : crospovidone, carmellose calcique, croscarmellose sodique, amidon de maïs et glycolate d'amidon de sodium.

9. Comprimé orodispersible selon l'une quelconque des revendications 5 à 7, comprenant en outre de la crospovidone.

10. Comprimé orodispersible selon l'une quelconque des revendications 5 à 9, où le comprimé se désintègre dans les 60 secondes dans « l'essai de désintégration » dans la pharmacopée japonaise 16^{ème} édition, dans laquelle le résultat est la valeur maximum de 6 comprimés.

11. Procédé de production d'un comprimé orodispersible, comprenant :
une étape de production de granulés contenant un médicament par soit :
le mélange (A) d'hydrate de tosylate d'edoxaban, (B) de 0,1 à 15 % en poids d'acide fumarique par rapport au poids total du comprimé et (E) de carmellose et la pulvérisation, sur le mélange, de (C) 0,1 à 2,0 % en poids d'hydroxypropylcellulose par rapport au poids total du comprimé, dissous dans de l'eau ; soit
le mélange (A) d'hydrate de tosylate d'edoxaban, (B) de 0,1 à 15 % en poids d'acide fumarique par rapport au poids total du comprimé, (C) de 0,1 à 2,0 % en poids d'hydroxypropylcellulose par rapport au poids total du comprimé et (E) de carmellose et la pulvérisation d'eau sur le mélange ;
une étape de production de granulés sans médicament par le mélange (F) de D-mannitol et (G) d'une cellulose microcristalline ayant une masse volumique en vrac de 0,26 g/cm³ ou moins et la pulvérisation, sur le mélange, (H) d'amidon prégélatinisé dissous ou dispersé dans de l'eau pour produire des granulés sans médicament ; et
une étape de moulage par compression des deux types de granulés obtenus.
